# EUROPÄISCHE PATENTSCHRIFT

(11) **EP 0 801 948 B1**
(45) Veröffentlichungstag und Bekanntmachung des Hinweises auf die Patenterteilung: **06.06.2001**
(21) Anmeldenummer: 97103736.1
(22) Anmeldetag: 06.03.1997
(51) Int. Cl.: A61K 9/00

(54) **Ophtalmische Zusammensetzung mit verlängerter Verweilzeit am Auge**
Ophthalmic composition having prolonged residence time in the eye
Composition ophtalmique à temps de sejour prolongé

(30) Priorität: 15.04.1996 DE 19614823
(43) Veröffentlichungstag der Anmeldung: 22.10.1997
(73) Patentinhaber: Dr. GERHARD MANN chem.-pharm. Fabrik GmbH, D-13581 Berlin (DE)
(72) Erfinder: Bellmann, Günther, Dr., 13593 Berlin (DE); Claus-Herz, Gudrun, Dr., 14167 Berlin (DE)
(74) Vertreter: Maiwald, Walter, Dr. Dipl.-Chem.

(56) Entgegenhaltungen:
- EP-A- 0 470 667
- FR-A- 2 678 832
- FR-A- 2 679 773
- VESTN. OFTAL'MOL., Nr. 2, 1976, MOSCOW (SU), Seiten 29-31, XP002034466 KONDRAT'EVA, T. S.; ET AL: "Study of the hypotensive and myotic effects of various pilocarpine solutions (experimental studies)"
- FARMATSIYA, Bd. 19, Nr. 1, 1970, MOSCOW (SU), Seiten 23-26, XP002034467 IVANOVA, L. A.; ET AL.: "Enhanced antimicrobial stability of ointment bases"
- ANN. ACAD. MED. SILESIENSIS, Nr. 23, 1991, KATOWICE (PL), Seiten 91-100, XP002034468 WOJDAK, HALINA; ET AL.: "Assessment of the effect of excipients upon the stability of Sefril and indomethacin in eye preparations"
- FALBE J et al., 'Römpp Chemie Lexikon', Seite 1511, 9. Auflage 1990, Georg Thieme Verlag, Stuttgart
- LIST, "Arzneiformenlehre", Seite 442, 4. Auflage, 1985, Wissenschaftliche Verlagsgesellschaft, Stuttgart

## Beschreibung

Die Erfindung betrifft die Verwendung eines Benzyllauryldimethylammoniumsalzes zur Herstellung von ophthalmischen Zusammensetzungen, die zur wiederholten Anwendung über längere Zeiträume bestimmt sind und/oder für eine längere Verweildauer am Auge nach jeder Anwendung formuliert sind, zur Vermeidung von durch Benzalkoniumchlorid hervorgerufenen Irritationen und/oder Gewebeschädigungen des Auges.

Ophthalmische Zusammensetzungen können, wie andere pharmazeutische Präparate auch, durch Mikroorganismen der unterschiedlichsten Art verunreinigt sein. Es muß vermieden werden, daß solche Mikroorganismen bei der Anwendung der ophthalmischen Zusammensetzung in das Auge und auf die Augenschleimhäute gelangen können. Ophthalmische Präparate müssen daher hohen Anforderungen an die Sterilität gerecht werden. Aus diesem Grund wird die Herstellung derartiger Präparate grundsätzlich unter Sterilbedingungen durchgeführt.

Um die Sterilität und insbesondere die Lagerfähigkeit von ophthalmischen Präparaten, die nicht zum sofortigen Gebrauch bestimmt sind, zu erhöhen, werden diesen Konservierungsmittel zugefügt. Diese müssen dabei einerseits eine hinreichend keimtötende Wirkung aufweisen, um die dauerhafte Sterilität des Präparates zu sichern, andererseits dürfen sie nicht selbst zu Irritationen oder Gewebeschädigungen des, häufig ja schon vorgeschädigten, Auges führen.

Besonders kritisch sind diese Anforderungen bei ophthalmischen Zusammensetzungen, die über längere Zeiträume (einen oder mehrere Tage und länger) wiederholt angewendet werden müssen, so daß sich andauernd ein Konservierungsmittel-Spiegel im Auge einstellt. Hier ist die Gefahr von Reizungen oder sogar Gewebeschädigungen durch das Konservierungsmittel besonders hoch.

Ähnliches gilt für solche ophthalmischen Zusammensetzungen, die nach einmaliger Anwendung eine (im Vergleich mit leicht durch Tränenflüssikeit auswaschbaren Präparaten, wie z.B. üblichen wäßrigen Tropf-Lösungen) verlängerte Verweildauer am bzw. im Auge zeigen. Solche Präparate sind hinsichtlich ihrer Konsistenz mit erhöhter Viskosität eingestellt. Ein Beispiel sind durch Zugabe wasserlöslicher Polymere gelartig verdickte wäßrige Präparate, insbesondere sogenannte tropfbare Gele. Ein anderes Beispiel sind die bekannten Salben, meist in Form von streichfähigen Emulsionen.

Als Konservierungsmittel sind für solche Zwecke z.B. Thiomersal, Centrimid, und dgl. Substanzen im Einsatz.

Ein anderes häufig vorgeschlagenes Konservierungsmittel für ophthalmische Präparate ist Benzalkoniumchlorid.

Benzalkoniumchlorid ist der internationale Freiname für N-alkyl-N-benzyl-N,N-dimethylammoniumchlorid, mit Alkylresten zwischen C₈H₁₇ und C₁₈H₃₇. Benzalkoniumchlorid wird üblicherweise aus natürlichen Fetten oder Ölen gewonnen und ist, in Abhängigkeit von den verwendeten Rohstoffen, ein Gemisch wechselnder Zusammensetzung der zuvor beschriebenen quaternären Verbindungen.

In dieser Anmeldung wird, soweit nichts anderes angegeben ist, mit dem Begriff "Benzalkonium" immer ein solches Gemisch mit unterschiedlichen Alkylresten gemeint, wobei die Anzahl der Kohlenstoffatome im Alkylrest von C₈ bis C₁₈ variiert.

In der US-Patentschrift US 40 53 628 wird eine klare Lösung von Natriumcromoglicat offenbart, die mit einer Vielzahl von Konservierungsmitteln versehen sein kann. Vorgeschlagen und exemplifiziert wird neben Thiomersal, Centrimid, Benzetheniumchlorid und anderen, auch Benzalkoniumchlorid. Die Möglichkeit, die Viskosität solcher Lösungen einzustellen, wird allgemein erwähnt; die Beispiele betreffen jedoch ausschließlich Lösungen ohne viskositätsändernde Zusätze. Die Gefahr von Augenreizungen durch das Konservierungsmittel wird in dieser Veröffentlichung nicht angesprochen.

Aus der US-Patentschrift US 42 71 143 ist es bereits bekannt, ein ophthalmisches Gel zur verzögerten Wirkstofffreisetzung mit Benzalkoniumchlorid zu konservieren. Über Augenreizungen bei der vorgesehenen langen Verweilzeit im Auge wird nicht berichtet. Die Versuche am Auge, die in der US 42 71 143 beschrieben werden, dauerten jeweils nur einige Stunden. Dies ist eventuell der Grund, warum die Probleme, die bei solchen Gelen in der längerdauernden Anwendung auftreten, anscheinend nicht beobachtet wurden.

Die französische Patentanmeldung FR-2 678 832 beschreibt ophthalmische Zusammensetzungen enthaltend verschiedenste Wirkstoffe und Polyacrylsäure als Verdickungsmittel zur Erzielung eines anhaltenden Wirkeffekts am Auge. Die FR-2 678 832 macht keine Angaben über die Verträglichkeit der verwendeten Konservierungsmittel, insbesondere nicht bei lang andauernder und wiederholter Anwendung.

Aus der französischen Patentanmeldung FR-2 679 773 sind ophthalmische Präparate in verschiedenen Applikationsformen bekannt, welche durch Zusatz eines osmotisch verträglichen antimikrobiellen Stoffes für das Auge verträglicher gemacht werden. Die beschriebenen verwendbaren Konservierungsmittel umfassen unter anderem auch Benzalkoniumchlorid, welches bei langandauernder oder wiederholter Anwendung die Augen reizt. Die Verwendung von Benzyllauryldimethylammoniumchlorid und dessen irritationsvermeidender Effekt wird in der FR-2 679 773 nicht erwähnt.

Benzalkoniumchlorid besitzt ausgezeichnete antiseptische Eigenschaften auch in ophthalmischen Präparationen, insbesondere in wäßrigen ophthalmischen Präparationen. Jedoch weist Benzalkoniumchlorid, wie sich seit Veröffentlichung der vorgenannten Patentschriften gezeigt hat, eine schlechte Verträglichkeit auf, die zu Irritationen und sogar Läsionen des Auges führen kann. B. Lopez et al. (Current Eye Research, 1991, 10 (7,645 bis 656) berichten über die schädigende Wirkung von Konservierungsmitteln in künstlichen Tränenflüssigkeiten auf die Hornhaut (Cornea) bei Kaninchen. Die Wirkung von Tränenflüssigkeiten, die mit 0,01% Benzalkoniumchlorid, 0,001% Polyquat oder 0,004% Thiomersal konserviert waren, wurde in Bezug gesetzt zu einer Vergleichsgruppe, bei der künstliche Tränenflüssigkeit ohne Konservierungsmittel verwendet wurde.

Als Maß für die Schädigung der Cornea wurde die Steigerung der Aufnahmefähigkeit für Carboxyfluorescein gewählt. Künstliche Tränen, die Polyquat oder Thiomersal enthielten, bewirkten eine bis zu vierfache Aufnahmesteigerung. Künstliche Tränen, die Benzalkoniumchlorid enthielten, bewirkten eine etwa 10 bis 100-fache Aufnahmesteigerung.

Elektronenmikroskopische Kontrolluntersuchungen belegten, daß die Steigerung der Aufnahmefähigkeit für Carboxyfluorescein mit einer verstärkten Zellschädigung in der Cornea einhergingen. Im Ergebnis wurde von der Verwendung von Benzalkoniumsalzen für Ophthalmica dringend abgeraten.

In jüngerer Vergangenheit hat sich das Interessse einzelnen Komponenten des Benzalkoniums zugewandt. So beschreibt die JP-A 1246227 ein Verfahren zur Vermeidung von Inkompatibilitäten in flüssigen wäßrigen ophthalmischen Zusammensetzungen, insbesondere Augen tropfen, die Benzalkoniumchlorid enthalten. Eine Vielzahl von medizinischen Wirkstoffen zeigt in der Darreichungsform als wäßrige Lösung eine Inkompatibilität mit Benzalkoniumchlorid, die zur Bildung und Ausflockung von löslichen Verbindungen führt. Eine Verwendung von Benzalkoniumchlorid als Konservierungsmittel ist in derartigen wäßrigen Zusammensetzungen daher nicht möglich. Bei Verwendung von nahezu reinem Benzyllauryldimethylammoniumchlorid, also dem C₁₂-Homologen aus dem Benzalkoniumchlorid-Gemisch treten die beschriebenen Inkompatibilitäten nicht auf. Eine Konservierung von Augentropfen, deren Wirkstoff inkompatibel mit Benzalkoniumchlorid wäre, ist somit mittels Benzyllauryldimethylammoniumchlorid möglich.

Dieser Stand der Technik betrifft nicht das Problem der mangelnden Augenverträglichkeit von Benzalkoniumchlorid.

Aufgabe der vorliegenden Erfindung ist es, eine ophthalmische Zusammensetzung der eingangs genannten Art bereitzustellen, die eine gute Verträglichkeit gegenüber dem Auge, auch bei andauernder Einwirkung, aufweist und die Nachteile des Standes der Technik überwindet, ohne dabei die vorteilhaften Wirkungen der Benzalkoniumsalze als Konservierungsmittel aufzugeben.

Die Lösung dieser Aufgabe wird erfindungsgemäß durch die Merkmale des unabhängigen Anspruchs ermöglicht.

Die abhängigen Ansprüche definieren vorteilhafte Ausgestaltungen der Erfindung.

Es wurde überraschend festgestellt, daß bei der Verwendung von Benzyllauryldimethylammonium-Salzen als Konservierungsmittel in ophthalmischen Präparaten deutlich weniger oder gar keine Irritationen und Läsionen des Auges auftreten, die jedoch bei Verwendung anderer Konservierungsmittel, einschließlich Benzalkoniumchlorid, beobachtet werden.

Die Erfindung macht es daher insbesondere möglich, solche ophthalmischen Zusammensetzungen zu konservieren, die eine im Vergleich mit Augentropfen oder dergleichen verlängerte Verweilzeit am Auge haben sollen. Insbesondere können so tropfbare Gele, Salben und dergleichen geschaffen werden, die nach einmaliger Anwendung sehr lange auf das Auge einwirken, weil sie nur langsam durch die Tränenflüssigkeit ausgewaschen werden, wobei das Konservierungsmittel alle Vorteile des bekannten Benzalkoniumchlorids aufweist, ohne aber zu dessen augenreizender oder sogar augenschädigender Wirkung zu führen.

Vorzugsweise handelt es sich bei den erfindungsgemäßen ophthalmischen Zusammensetzungen um solche, die als Träger eine wäßrige, tropfbare Gelbasis aufweisen. Dabei wird man in bekannter Weise ein viskositätserhöhendes synthetisches oder natürliches Polymer in wäßriger Lösung oder wäßriger Dispersion einsetzen.

Hierfür eignen sich insbesondere die als Gelbildner bereits bekannten Carboxyvinylpolymere, insbesondere Carboxypolymethylene, die unter der Marke "Carbopol" im Handel erhältlich sind. Alternativ lassen sich die unter der Marke "EMA" im Handel erhältlichen Ethylen-Maleinsäureanhydrid-Copolymere einsetzen.

An natürlichen Polymeren eignen sich insbesondere die diversen, wiederum für ophthalmische Gele bereits bekannten Cellulosederivate, insbesondere Alkylcellulosen, Hydroxycellulosen, Hydroxyalkylcellulosen, etc.. Mit Vorteil lassen sich zusätzlich oder alternativ natürliche Gummis verwenden, wie beispielsweise Guar-Gummi, Xanthan-Gummi, usw.. Weitere Beispiele für erfindungsgemäß vorteilhaft verwendbare natürliche Polymere sind Dextran und seine Derivate.

Es kann vorteilhaft sein, die erfindungsgemäße ophthalmische Zusammensetzung als grundsätzlich einphasige wäßrige Flüssigkeit zu formulieren, in der die weiteren Bestandteile gelöst oder als dispergierte Teilchen auftreten.

Alternativ und oft noch vorteilhafter kann die Zusammensetzung als zweiphasige Flüssigkeit aufgebaut werden, mit einer wäßrigen und einer hydrophoben Phase. Besonders wenn das Präparat bestimmte Wirkstoffe, wie etwa Vitamin A enthält, wird man es vorziehen, eine kontinuierliche wäßrige Phase mit darin emulgierten Tröpfchen der hydrophoben Phase vorzusehen. Als hydrophobe Phase eignen sich Öle, mittelkettige Triglyceride, usw.. Insbesondere bei Präparaten, die als Wirkstoff Vitamin A enthalten, wird man mit Vorteil mittelkettige Triglyceride als hydrophobe Phase anwenden.

Die Konzentration des Benzyllauryldimethylammonium-Salzes entspricht den üblichen Konzentrationen, wie sie für Benzalkoniumchlorid angesetzt werden. Das Benzyllauryldimethylammonium-Salz ist vorzugsweise einfach das Chlorid.

Neben den soeben beschriebenen ophthalmischen Zusammensetzungen, die für eine längere Verweildauer am Auge nach jeder Anwendung formuliert wurden, läßt sich der Vorteil der Erfindung auch für solche ophthalmischen Zusammensetzungen nutzen, die zwar keine viskositätserhöhenden (und damit das Auswaschen verzögernden) Bestandteile enthalten, aber über längere Zeiträume wiederholt angewendet werden müssen und so ebenfalls zu einem dauernd erhöhten Konservierungsmittel-Spiegel im Auge führen. Auch solche Präparate können, wenn schädigende Konservierungsmittel enthalten sind, zu Irritationen oder sogar Gewebeschädigungen führen und daher läßt sich auch für solche Präparate der erfindungsgemäße Vorteil nutzen, indem statt dieser Konservierungsmittel das Benzyllauryldimethylammonium-Salz als Konservierungsmittel eingesetzt werden.

Beispiele hierfür sind nicht nur Augentropfen, sondern auch künstliche Tränenflüssigkeit, wie bei dem von Lopez et al berichteten Versuch.

Die folgenden Ausführungsbeispiele dienen nur der Erläuterung der Erfindung und stellen keinerlei Einschränkung dar.

### Beispiel 1

Ophthalmische Zusammensetzung mit 0,01% Benzyllauryldimethylammoniumchlorid.
Ansatzgröße: 2 kg

| Bestandteile | Menge in Gramm |
|---|---|
| Carbopol 980 NF | 4,00 |
| Benzyllauryldimethylammoniumchlorid | 0,2000 |
| Sorbitol | 80,00 |
| NaOH, fest | 1,57 |
| Wasser | (Rest bis Ansatzgröße) |

### Beispiel 2

Ophthalmische Zusammensetzung mit 0,005% Benzyllauryldimethylammoniumchlorid.
Ansatzgröße: 2 kg

| Bestandteile | Menge in Gramm |
|---|---|
| Carbopol 980 NF | 4,00 |
| Benzyllauryldimethylammoniumchlorid | 0,1000 |
| Sorbitol | 80,00 |
| NaOH, fest | 1,57 |
| Wasser | (Rest bis Ansatzgröße) |

Mit den Zusammensetzungen gemäß Beispiel 1 und 2 wurden Langzeittests an Versuchskaninchen (Charles River) durchgeführt. Parallel wurden mit einer entsprechenden Kontrollgruppe Vergleichsversuche durchgeführt, bei denen in jeder Hinsicht gleiche Zusammensetzungen eingesetzt wurden, die sich jedoch hinsichtlich des Konservierungsmittels unterschieden. Statt Benzyllauryldimethylammoniumchlorid wurde einerseits Benzalkoniumchlorid in gleicher Konzentration, andererseits Thiomersal (Standardkonzentration) = 40 *µ*m/g verwendet.

Die Versuchsdauer betrug 5 Wochen.

Am Ende des Versuchs zeigten sieben von acht Kaninchen der Kontrollgruppe, die mit der Thiomersal-konservierten Zusammensetzung behandelt worden war, starke Irritationen, teilweise auch Läsionen am Auge.

Bei der Kontrollgruppe, die ein mit Benzalkoniumchlorid konserviertes Präparat erhielt, zeigten alle Kaninchen am Ende der Untersuchung starke Irritationen, teilweise Gewebeschädigungen am Auge.

Hingegen führten die ophthalmischen Zusammensetzungen gemäß den erfindungsgemäßen Beispielen 1 und 2 bei keinem Kaninchen am Ende der Versuchsdauer zu einer feststellbaren Irritation des Auges. Ebenfalls wurden keinerlei Gewebeschädigungen beobachtet.

## Patentansprüche

1. Verwendung eines Benzyllauryldimethylammonium-Salzes als Konservierungsmittel zur Herstellung ophthalmischer Zusammensetzungen, die zur wiederholten Anwendung über längere Zeiträume bestimmt sind und/oder für eine längere Verweildauer am Auge nach jeder Anwendung formuliert sind, zur Vermeidung von durch Benzalkoniumchlorid hervorgerufenen Irritationen und/oder Gewebeschädigungen des Auges.

2. Verwendung von Benzyllauryldimethylammoniumchlorid gemäß Anspruch 1.

3. Verwendung eines Konservierungsmittels gemäß Anspruch 1 oder 2 zur Herstellung von mit wenigstens einem Wirkstoff versehenen Augentropfen.

4. Verwendung des Konservierungsmittels gemäß Anspruch 1 oder 2 zur Herstellung einer künstlichen Tränenflüssigkeit.

5. Verwendung einer ophthalmischen Zusammensetzung zur verlängerten Verweilzeit am Auge in Form eines tropfbaren Gels enthaltend 0,001 bis 1 Gew.-%, vorzugsweise 0,1 bis 0,5 Gew.-% Carboxypolymethylen, 0,0005 bis 0,05 Gew.-%, bevorzugt 0,001 bis 0,01 Gew.-% Benzyllauryldimethylammoniumchlorid, 0,1 bis 10 Gew.-%, bevorzugt 1 bis 5 Gew.-% Sorbitol, sowie Alkalihydroxid bzw. Säure zur Einstellung eines physiologisch akzeptablen pH-Wertes und im übrigen Wasser, zur Vermeidung von durch Benzalkoniumchlorid hervorgerufenen Irritationen und/oder Gewebeschädigungen des Auges.

## Claims

1. Use of a benzyl lauryl dimethyl ammonium salt as a preservative for the manufacture of ophthalmic preparations, which are intended for repeated application over prolonged periods of time and/or formulated for a prolonged residence time at the eye after each administration, to avoid irritations and/or tissue damages of the eye caused by benzalconium chloride.

2. Use of benzyl lauryl dimethyl ammonium chloride according to claim 1.

3. Use of a preservative agent according to claim 1 or 2 for the manufacture of eye drops comprising at least one active agent.

4. Use of a preservative according to claim 1 or 2 for the manufacture of an artificial tear liquid.

5. Use of an ophthalmic preparation for prolonged residence time on the eye in the form of a gel applicable in drop form comprising 0.001 to 1 weight percent, preferably 0.1 to 0.5 weight percent of carboxy polymethylene, 0.0005 to 0.05 weight percent, preferably 0.001 to 0.01 weight percent of benzyl lauryl dimethyl ammonium chloride, 0.1 to 10 weight percent, preferably 1 to 5 weight percent of sorbitol, and alkaline hydride or acid, respectively, for adjusting a physiologically acceptable pH value and water for the rest, to avoid irritations and/or tissue damages of the eye caused by benzalconium chloride.

## Revendications

1. Utilisation d'un sel de benzyllauryldiméthylammonium en tant que conservateur pour la production de compositions ophtalmiques destinées à des applications répétées pendant un temps assez long et/ou formulées pour un temps de séjour relativement long sur l'oeil après chaque application, en vue d'éviter les irritations et/ou lésions des tissus oculaires provoquées par le chlorure de benzalkonium.

2. Utilisation de chlorure de benzyllauryldiméthylammonium selon la revendication 1.

3. Utilisation d'un conservateur selon la revendication 1 ou 2 pour produire des collyres comportant au moins un principe actif.

4. Utilisation du conservateur selon la revendication 1 ou 2 pour produire une solution de larmes artificielles.

5. Utilisation d'une composition ophtalmique à temps de séjour prolongé sur l'oeil sous la forme d'un gel instillable contenant 0,001 à 1 % en poids, de préférence 0,1 à 0,5 % en poids de carboxypolyméthylène, 0,0005 à 0,05 % en poids, de préférence 0,001 à 0,01 % en poids de chlorure de benzyllauryldiméthylammonium, 0,1 à 10 % en poids, de préférence 1 à 5 % en poids de sorbitol, ainsi qu'un hydroxyde alcalin ou un acide, pour obtenir un pH physiologiquement acceptable et, en tant qu'excipient, de l'eau, pour éviter les irritations et/ou lésions des tissus oculaires provoquées par le chlorure de benzalkonium.
